# EUROPEAN PATENT SPECIFICATION

(11) **EP 2 405 848 B1**
(45) Date of publication and mention of the grant of the patent: **03.08.2016**
(21) Application number: 10751340.0
(22) Date of filing: 10.03.2010
(51) Int. Cl.: A61L 31/10, A61L 31/16

(54) **POWDER-FREE ANTI-BLOCKING COATED GLOVE**
PULVERFREIER HANDSCHUH MIT ANTIBLOCKBESCHICHTUNG
GANT REVÊTU ANTI-ADHÉRENT EXEMPT DE POUDRE

(30) Priority: 11.03.2009 US 159266 P; 09.03.2010 US 720155
(43) Date of publication of application: 18.01.2012
(73) Proprietor: Ansell Limited, Red Bank, NJ 07701 (US)
(72) Inventor: ENG, Aik Hwee, Petaling Jaya 47810 (MY); LAI, Hee Meng, Melaka (MY); LIM, Kuang Leng, Selangor 41050 (MY); TING, Koon Meow, Selangor 42100 (MY); LUCAS, David M., Petaling Jaya Selangor 47410 (MY); NARASIMHAN, Dave, Flemington NJ 08822 (US)
(74) Representative: Zimmermann & Partner Patentanwälte mbB
(86) International application number: PCT/US2010/026793
(87) International publication number: WO 2010/104931

(56) References cited:
- EP-A2- 0 300 814
- US-A- 5 284 607
- US-A- 5 357 636
- US-A- 6 019 922
- US-A1- 2004 091 504
- US-A1- 2004 115 250
- US-B1- 6 195 805
- US-B1- 6 347 408

## Description

### TECHNICAL FIELD

The present invention generally relates to elastomeric articles, such as gloves, particularly gloves for use in the medical profession that has a liquid or gel-based coating that is protected with an anti-blocking surface film.

### BACKGROUND

Skin-contacting articles, such as gloves, particularly medical gloves, are commonly used as a protective barrier against the contamination of the user by chemicals and body fluids containing microorganisms including bacteria and/or viruses and the like. As such, these gloves and other skin-contacting articles are manufactured in such a way that they are entirely impermeable to the contaminants or microorganisms during use. However, medical gloves are typically extremely thin, manufactured from soft elastomeric materials such as natural or synthetic rubbers and may puncture or rupture during use, allowing microorganisms to pass through the discontinuity in the glove. In addition, repeated and prolonged wearing of gloves warrants the need to offer gloves that provide comfort and ease of use.

One approach to these needs has been the application of liquid or gel-based coatings to the elastomeric articles. However, these coatings cause the inner surface of the gloves to stick together by a phenomenon termed as 'blocking' and an anti-blocking coating is needed over the liquid or gel coating to ensure easy donning of the glove by the user.

For example, U.S. Patent No. 4,853,978 (Stockum) discloses a natural rubber surgical glove having an inner coating containing the cationic antimicrobial agents chlorhexidine gluconate (CHG) or polyhexamethylene biguanide hydrochloride (PHMB). Cross-linked cornstarch is included in the inner coating as an anti-blocking agent, which is also said to affix the CHG and PHMB in place and allow their slow release during perspiration. However, the use of cross-linked cornstarch is not compatible with the trend towards powder-free medical environments.

U.S. Patent 5,089,205 (Huang) discloses natural latex gloves having an inner lubricant layer containing chlorhexidine diacetate. Again, powdered starch is disclosed as an anti-blocking agent. Although the patent teaches modifying the chlorhexidine with an anionic or nonionic surfactant to render it compatible with anionic components of the gloves, no mention is made of the effect of the surfactant on antimicrobial efficacy since the anionic or non-ionic surfactant (which has both anionic and cationic counter parts that have equivalent charges) will clearly neutralize the chlorhexidine cationic antimicrobial agent.

U.S. Patent No. 5,133,090 (Modak et al.) discloses a latex glove having an inner coating of chlorhexidine and a lubricating agent, such as zinc oxide, hydroxycellulose or corn starch. Corn starch can be used provided it has been modified with a surfactant to block adsorption sites for the chlorhexidine. Again use of corn starch does not result in a powder free surgical glove.

U.S. Patent No. 5,483,697 (Fuchs) discloses a double-layer latex glove having an inner layer, an outer layer sealing a middle layer comprising an antimicrobial agent in the form of a solution, which may be mixed with a hydrogel. The hydrogel is said to physically block or plug holes that form in the inner and outer layers. Examples of hydrogels include corn starch and derivatives of cellulose. No mention is made of the effect of the hydrogel on the efficacy of the antimicrobial agent. The antimicrobial agent is not delivered to the hand of the user killing any germs present, rather guards against any microbes present in a sharp instrument when the outer surface of the glove is breached. Clearly, the kill time of the microbes becomes an extremely important factor to produce effective kill since the outer glove breach occurs in a short time frame.

U.S. Patent No. 5,570,475 (Nile et al.) discloses a surgeon's glove having a polymeric donning layer disposed thereon comprising a polymer of specific repeating units. The polymeric layer preferably has raised domains. No mention is made of any liquid or gel coating on the interior surface of the glove.

U.S. Patent No. 6,391,409 (Yeh et al.) discloses a powder free nitrile-coated natural rubber glove having an intermediate layer comprised of a synthetic rubber and nitrile rubber. The intermediate layer is said to aid donning and prevent self-sticking of the inner glove surfaces. No mention is made of any liquid or gel coating on the interior surface of the glove.

U.S. Patent No. 6,378,137 (Hassan et al) discloses a powder-free polymeric medical glove having an outer side silicone-treated surface and an inner side bonded to anti-blocking layer formed from a polymer/copolymer, a high density polyethylene particle, and a wax, the anti-blocking layer coated with a layer of silicone emulsion. Silicone used may be a liquid and is thus subject to blocking. Use of wax may prevent the uniform distribution of the liquid layer on the interior surface of the glove as a thin film.

U.S. Patent No. 7,032,251 (Janssen) discloses an elastomeric article having a donning layer comprising a polymeric coating cross-linked with a polyamine epichlorohydrin crosslinking agent. The polymeric coating may comprise cellulose derivatives. No mention is made of any liquid or gel coating on the interior surface of the glove.

U. S. Patent Publication No. 2003/0204893 (Wang et al.) discloses an elastomeric glove having a preparation disposed on the interior surface of the glove comprising an acidic antimicrobial substance and buffer that resists pH change. The preparation may also comprise thickeners, which include cellulose derivatives. No mention is made of the tackiness or blocking properties of the preparation. The thickener used is dissolved in the composition and does not form a protective film. Besides, the coating is not indicated to be a liquid coating or a gel coating on the interior surfaces of the glove.

U. S. Patent Publication No. 2004/0115250 (Loo et al.) discloses an elastomeric glove having a moisturizing film comprising glycerol and a botanical extract, such as aloe vera and chamomile. The film may also comprise an anti-blocking wax. The glycerol containing moisturizing dried layer comprises anti-blocking wax and is not protected by a film.

U.S. Patent Publication No. 2005/0081278 (Williams) discloses an elastomeric glove having a dried lotion coating on the inside skin-contacting surface comprising a film-forming compound and an oil-based emollient. Film forming compounds include polyurethane, acrylonitrile, neoprene, acrylic latex styrene butadiene rubber and polisoprenecal. Antimicrobial agents may be present in the coating, but only in amounts sufficient to act as preservatives. No mention is made of the tackiness of the coating, which will lead to blocking of the glove interior surfaces. The lotion coating is not protected by an anti-blocking film.

U.S. Patent Publication No. 2005/0112180 (Chou) discloses an elastomeric glove having an external first layer comprising an antimicrobial agent and an internal second hand-contacting layer configured to resist penetration by the antimicrobial agent. The interior surface of second layer may be provided with an acidic preparation comprising one or more of a second antimicrobial agent, a buffer, a moisturizer, a soothing agent and a thickener. No mention is made of the tackiness of the preparation applied to the second layer.

U.S. Patent Publication No. 2006/0059604 (Lai et al.) and U.S. Patent No. 6,709,725 (Lai et al.) disclose latex gloves coated with a non-tacky aqueous polymeric emulsion comprising a film-forming polymer or copolymer, a wax, a surfactant and a hardness modifier. There is no gel coating on the glove interior surface and there is no protective film present over a gel coating.

U.S. Patent Publication No. 2006/0070167 (Eng et al.) discloses a rubber glove having a dried coating of an emulsified hand-friendly mixture comprising a water-soluble humectant moisturizer, a water-insoluble occlusive moisturizer, a water-soluble surfactant, and a water-soluble lubricant. The coating mixture may also contain an antimicrobial agent. The coating is said to be inherently non-tacky and non-blocking due to the fine dispersion of the water insoluble occlusive moisturizer within the dried coating and the coating is said to improve donning of the glove. Even the coating contains water insoluble occlusive moisture that has a potential for blocking or adhering the interior glove surfaces, the fine dispersion of the occlusive moisturizer in the dried coating avoids the presence of large areas of the water insoluble occlusive moisture. There is no protective film present over a gel coating.

U.S. Patent Publication No. 2007/0104766 (Wang et al.) discloses a powder-free elastomeric article having a surface coating comprising an antimicrobial agent, hydrophilic film-forming polymer and a hydrophobic component. The non-volatile water-soluble antimicrobial agent is incorporated in a controlled-release matrix comprising a blend of a hydrophilic polymer and a hydrophobic component. The controlled-release matrix/ blend requirements include: compatibility with the antimicrobial agent, formation of a reservoir of antimicrobial agent, coating film flexibility and lower water-solubility of the matrix/ blend than that of the antimicrobial agent. The hydrophilic polymers include cellulosic polymers. The hydrophilic polymer is believed to provide a reservoir for the antimicrobial agent, while the hydrophobic component is believed to improve the film's flexibility through its plasticizing effect. The balance of hydrophilicity and hydrophobicity in the coating film is said to control the release of the antimicrobial agent. The hydrophilic cellulosic polymer is not provided as a film protecting a gel coating.

EP300814 discloses a powder-free medical glove made of elastomeric material containing an inner coating comprising an antimicrobial agent. In order to be powder-free, the inner surface of the gloves has been surface modified to allow donning. The surface modification consists of the application of a low coefficient of friction elastomer coating to the inside of the gloves.

Thus, there remains a need for powder-free elastomeric articles, such as medical gloves, which exhibit good anti-blocking properties even when a liquid- or gel-based coating is applied on the interior surfaces of the glove.

### SUMMARY

The present invention provides powder-free elastomeric articles exhibiting anti-blocking properties in combination with excellent therapeutic properties, providing one or more beneficial effects, for example, skin-nourishing compositions, warming or cooling sensations, and/or fragrances. The therapeutic properties are provided by a fluid coating, that is, a coating that is not solid and can comprise one or more gels and/or liquids. The fluid coating is applied to the interior surface of the glove, which if left unprotected without further treatment, would subject the glove to blocking, wherein the interior surfaces of the glove stick together preventing easy donning of the glove. The interior surface of the glove having the fluid coating is covered with a cellulose-based solid film that seals the fluid coating preventing blocking of the glove. The cellulose-based solid film is capable of being dissolved easily by skin-generated moisture due to the unique properties of the cellulose-based containment film selected. Also provided herein are methods for the manufacture of such articles being durable and substantially free from interfering materials that could inactivate the therapeutic properties. In addition, packaging methods for maintaining efficacy of such therapeutic properties along with anti-blocking efficacy of the glove are provided.

Accordingly, one aspect of the present invention is directed to a powder-free elastomeric article, comprising:
an elastomeric layer having an interior surface and an exterior surface;
a therapeutic fluid coating applied to the interior surface of the elastomeric layer; and
an anti-blocking film coating disposed onto the therapeutic fluid coating.

Generally, the fluid coating comprises a formulation having both water soluble and water insoluble ingredients. For example, ingredients can include oily fragrances, and skin-conditioning agents such as silicones. The fragrances used may be water-based or oil-based and may not be completely dried especially when they are oil-based. The skin conditioning agents may include humectant moisturizers such as glycerol, polyvinyl alcohol, urea, emollients such as polydimethylsiloxane or dimethicone, oleyl erucate, lubricants such as polyethylene oxide and copolymer of polyethylene glycol and polypropylene glycol as disclosed in U.S. Patent Publication No. 2006/0070167 (Eng et al.) The Eng composition is generally formed from an aqueous solution that is substantially removed during processing. In a detailed embodiment, the aqueous solution comprises, in weight %, DC2-1352 Silicone Emulsion (water-insoluble occlusive moisturizer) in an amount of 1.20%, Polyox WSR N60K (lubricant) in an amount of 0.50%, Glycerol (water-soluble humectant) in an amount of 5.00%, Sodium lactate (water-soluble humectant) in an amount of 1.00%, Teric N100 (surfactant) in an amount of 2.30%, and in an amount of water 90.0%. This composition could stay in the liquid state after drying due to the presence of the silicone emulsion and/or high percentage of glycerol content and other components. Thus, these compositions are generally gel-like even in the dried condition on the internal surfaces of a glove, and thus, if left uncoated the gloves would become blocked, making donnability poor. Blocking can be prevented by the presence of the anti-blocking film of the subject invention.

Generally, the anti-blocking film coating comprises a cellulose composition that exhibits inverse solubility versus temperature. That is, these cellulose compositions dissolve readily in cold water but form a liquid crystal gel network when exposed to a higher temperature forming a two-phase structure. The gel formed is extremely stable at high temperatures. The transition temperature is called 'lower critical solution temperature' or LCST. Cellulose compounds that exhibit this behavior include methyl cellulose, hydroxypropylcellulose, hydroxypropyl methyl cellulose (HPMC) and hydroxyethyl methyl cellulose (HEMC). The LCST temperature depends on concentration in solution and the molecular weight of the composition. Higher molecular weight has a higher level of gel formation and lower solubility in cold water. The solution containing a dissolved LCST compound is applied using a dipping process or sprayed on the skin contacting surface of the glove with the gel coating and tumble dried or dired in drying tunnels at a temperature significantly greater than the LCST temperature. During the final drying process, the LCST film becomes a solid film covering the gel glove coating providing anti-blocking properties. The solid film formed will readily dissolve or disintegrate by the skin-generated moisture since the skin temperature is below the lower critical solution temperature LCST. This dissolution immediately releases the glove coating. The film strength may be improved by additives such as dicalcium phosphate dihydrate. In an embodiment, the anti-blocking film coating optionally comprises a water-insoluble occlusive moisturizer.

The article can be packaged in such a way as to preserve its fragrant or skin nourishing gel properties as well as anti-blocking behavior of the glove during transportation and storage. In an embodiment, the article is packaged in an inner wrap surrounded by an outer pack. The size of the inner wrap, after folding, is generally smaller than the outer pack for easy sealing. The pack is sealed such that a sufficient volume of dry air is present to provide a cushioning effect to the inner wrap containing the article. This cushioning air layer prevents undue pressure on the solid film that is covering the fragrant or skin nourishing gel coating on the interior skin contacting surface of the glove.

Another aspect of the present invention is directed to a process for preparing a powder-free therapeutic elastomeric article, comprising:
providing an elastomeric layer having an interior surface and an exterior surface;
applying a therapeutic fluid coating to the interior surface of the elastomeric layer; and
drying the therapeutic fluid coating; and
applying an anti-blocking film coating to the therapeutic fluid coating.

A detailed embodiment provides that the resulting gloves exhibit no loss of therapeutic properties following aging at 50° C for 8 weeks and 40°C for 6 months.

### DETAILED DESCRIPTION

Before describing several exemplary embodiments of the invention, it is to be understood that the invention is not limited to the details of construction or process steps set forth in the following description. The invention is capable of other embodiments and of being practiced or being carried out in various ways.

The present invention provides powder-free elastomeric articles exhibiting good therapeutic properties as well as anti-blocking properties. Applicants have surprisingly found that application of an anti-blocking coating to therapeutic coating in a fluid state such as a liquid or gel on an elastomeric glove not only prevents blocking, but there is no interference with the therapeutic properties during use and these properties are maintained during storage.

Accordingly, one aspect of the present invention is direct to a powder-free elastomeric article, comprising:
an elastomeric layer having an interior surface and an exterior surface;
an a therapeutic fluid coating applied to the interior surface of the elastomeric layer; and
an anti-blocking film coating applied to therapeutic fluid coating.

The gloves provide stable therapeutic properties, such as fragrance or skin-nourishment, meaning that these properties remain stable with no or little deterioration after the glove has been stored over the period of shelf-life of the glove. This can be indicated in an accelerated aging test, i.e. gloves exhibit no loss of fragrance or skin nourishing properties, following aging at 50° C for 8 weeks and 40°C for 6 months. The gloves also exhibit anti-blocking behavior after aging.

In a specific embodiment, the anti-blocking film coating comprises a cellulosic water-soluble polymer; and the drying step comprises: drying the anti-blocking film coating at a temperature greater than the low critical solution temperature of the cellulosic water-soluble polymer to form a liquid film, and further drying of the liquid film to form a solid film covering the therapeutic fluid coating.

By "powder-free" is meant an elastomeric article that has little or no powder or cross-linked starch. According to ASTM D 6124-01, the glove must have less than about 2 mg of powder or "any water insoluble, filter retained residue" per article.

By "interior surface" is meant the surface of the article that contacts the wearer. By "exterior surface" is meant the surface that contacts the atmosphere.

The elastomeric article can be any article intended to contact human skin, such as medical articles, including gloves, condoms, finger stalls, and the like. Latex used to prepare the articles can be provided in aqueous emulsions or solvent-based emulsions.

Suitable elastomers for use in the elastomeric layer include natural rubber latexes, such as Hevea and Guayule latexes, as well as synthetic latexes, such as polychloroprene, carboxylated acrylonitile butadiene, polyisoprene, polyurethane, styrene-butadiene, and the like. Blends of natural and synthetic latexes may also be used.

The elastomeric layer can be provided with a textured inner surface having micro-roughness for enhanced donning characteristics. The elastomeric layer can also be provided, alone or in conjunction with the textured inner surface, with a textured outer surface for enhanced grip characteristics. Methods for forming such textured elastomeric surfaces are provided in U.S. Patent Application Publication Nos. 2005/0035493 (Flather), 2007/0118967 (Flather), and 2006/0150300 (Hassan).

Since latex has a strong smell, it may be desirable to have a fragrance present on the skin-contacting surface of the glove. Such fragrance can prevent hands that have been in contact with the latex for a period of time from smelling like rubber, which can be offensive to many users. The fragrances employed may be of several known compositions, which may be water-based or oil-based. Generally these compositions may not dry completely since the volatile components of the fragrance volatilize long before the composition dries and usually have to be maintained in a wet state. If the coating with a fragrance is applied and is wet, it will readily block, making donning of the glove very difficult. The use of the anti-blocking coating of the present invention seals the liquid-containing fragrance, thereby preventing blocking of the glove and providing easy donnability.

Compositions that are nourishing to the hand are another form of a therapeutic coating. U.S. Patent Publication No. 2006/0070167 (Eng et al.) discloses one such coating. Skin-nourishing compositions can contain a lotion, a liquid gel or more commonly a silicone composition such as dimethicone. The liquid gels do not dry completely and are subject to stickiness or blocking of the inner surfaces of the glove preventing easy donnability. The use of the anti-blocking coating of the present invention seals the liquid-containing compositions, preventing blocking of the glove and providing easy donnability.

The anti-blocking coating applied to the gel coating can be any coating that prevents or inhibits the tackiness normally associated with the gel coating. Moreover, the nature of the coating is such that it is flexible enough not to flake or crack during storage despite its presence on an elastomeric material. When the article is a glove, the anti-block coating prevents blocking (sticking) of the interior of the gloves. In specific embodiments of the present invention, the anti-blocking coating is comprised of one or more cellulosic polymers, that are water-soluble, including hydroxypropylcellulose (HPC), hydroxypropylmethylcellulose (HPMC), methylcellulose (MC), and the like. The anti-block coating may additionally comprise other film-forming, water-soluble polymers such as polyvinylpyrrolidinone (PVP) and the like. In a detailed embodiment, the aqueous solution of anti-blocking coating comprises, in weight %, 1-10% HPC and 1-5% aminofunctional siloxane, or 0.1-5% HPMC

The preferred cellulose compositions are hydroxypropylcellulose (HPC), hydroxypropylmethylcellulose (HPMC) and methylcellulose (MC) since they exhibit inverse solubility phenomenon wherein the compound is readily soluble in water at temperatures below low critical solution temperature, LCST and form a liquid crystal gel network at temperatures above LCST. The gel stability only increases as the temperature is increased. As a result, the drying of the gel with protective LCST gel film results in progressively stronger gel that cures to a solid protective film. The LCST for HPC is 40 to 44°C. The LCST of HPMC can be reduced from this 40 to 44°C range by the degree of methylation as detailed in WO/1998/029501. The anti-blocking coating can optionally further comprise a water-insoluble occlusive moisturizer that can include, but is not limited to, aminofunctional siloxane, polydimethylsiloxane (dimethicone), oleyl erucate, or combinations thereof.

Hydroxypropylcellulose HPC is an ether of cellulose in which some of the hydroxyl groups in the repeating glucose units have been hydroxypropylated forming - OCH₂CH(OH)CH₃ groups. The average number of substituted hydroxyl groups per glucose unit is referred to as the degree of substitution (DS). Complete substitution would provide a DS of 3.

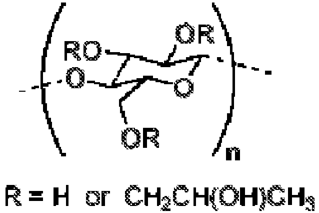

Because the hydroxypropyl group added contains a hydroxyl group, this can also be etherified during preparation of HPC. When this occurs, the number of moles of hydroxypropyl groups per glucose ring, moles of substitution (MS), can be higher than 3. Because cellulose is very crystalline, HPC must have an MS about 4 in order to reach a good solubility in water. HPC has a combination of hydrophobic and hydrophilic groups, so it has a lower critical solution temperature (LCST) at 45 °C. At temperatures below the LCST, HPC is readily soluble in water; above the LCST, HPC is not soluble. HPC forms liquid crystals and many mesophases according to its concentration in water. Such mesophases include isotropic, anisotropic, nematic and cholesteric.

Hydroxypropyl methylcellulose (HPMC) is a solid, and is a slightly off-white to beige powder in appearance and may be formed into granules. The compound forms colloids when dissolved in water and exhibits a thermal gelation property. That is, when the solution heats up to a critical temperature, the solution congeals into a non-flowable but semi-flexible mass. Typically, this critical (congealing) temperature is inversely related to both the solution concentration of HPMC and the concentration of the methoxy group within the HPMC molecule (which in turn depends on both the degree of substitution of the methoxy group and the molar substitution.

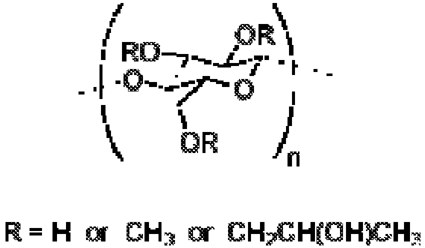

That is, the higher is the concentration of the methoxy group, the lower is the critical temperature. The inflexibility/viscosity of the resulting mass, however, is directly related to the concentration of the methoxy group (the higher is the concentration, the more viscous or less flexible is the resulting mass).

Methyl cellulose (MC) is a chemical compound derived from cellulose. It is a hydrophilic white powder in pure form and dissolves in cold (but not in hot) water, forming a clear viscous solution or gel.

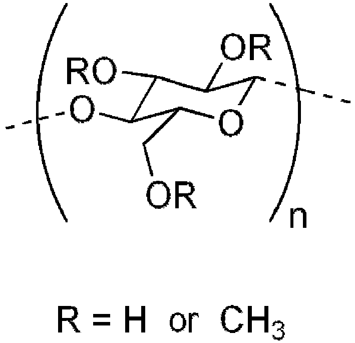

Chemically, methyl cellulose is a methyl ether of cellulose, arising from substituting the hydrogen atoms of some of cellulose's hydroxyl groups -OH with methyl groups -CH₃, forming -OCH₃ groups. Different kinds of methyl cellulose can be prepared depending on the number of hydroxyl groups so substituted. Cellulose is a polymer consisting of numerous linked glucose molecules, each of which exposes three hydroxyl groups. The Degree of Substitution (DS) of a given form of methyl cellulose is defined as the average number of substituted hydroxyl groups per glucose. The theoretical maximum is thus a DS of 3.0, however more typical values are 1.3 - 2.6. Different methyl cellulose preparations can also differ in the average length of their polymer backbones. Methyl cellulose dissolves in cold water. Higher DS-values result in lower solubility, because the polar hydroxyl groups are masked. The chemical is not soluble in hot water, which has the paradoxical effect that heating a saturated solution of methyl cellulose will turn it solid, because methyl cellulose will precipitate out. The temperature at which this occurs depends on DS-value, with higher DS-values giving lower precipitation temperatures.

Without being bound by any particular theory, it is believed that the therapeutic agents in the fluid coating migrate from the interior surface of the article to the user's skin upon hydration and/or rubbing action of the anti-blocking coating, thereby providing therapeutic benefits to the user. Following removal of the article, the coatings can provide a soft and smooth feel to the user's skin.

In one or more embodiments, the anti-blocking agents, fragrances and/or therapeutic fluids described herein may be provided in an encapsulated form or surrounded by a capsule or coating. Various encapsulation processes for forming a capsule or coating around substances have been developed and include processes which encapsulate ingredients by physical methods and/or chemical methods. Processes for encapsulating water soluble ingredients using a water insoluble capsule or hydrophobic shell are also known in the art. For example, U.S. Patent No. 6,890,653, describes microcapsule dispersions having a capsule core comprising water-soluble organic substances and a capsule coating of polyurethane and/or polyurea in a hydrophobic solvent. U.S. Patent No. 5,827,531 describes a process for forming multi-layered microcapsules that include alternating hydrophilic and hydrophobic liquid layers, surrounded by flexible, semi-permeable hydrophobic, outer membranes. One or more of the processes described in U.S. Patent No. 5,827,531 rely on low shear mixing and liquid-liquid diffusion process. An alternative process is described in U.S. Patent No. 4,102,806, which describes encapsulating core material which may be water-soluble or water-insoluble with an oleaginous material or "oil-and-fat" in an organic solvent. US Publication No. 2004/0048771 discloses an interfacial polymerization process to encapsulate an ingredient with an encapsulation wall made from a water-insoluble urea-formaldehyde polymer.

Other processes for encapsulating water-insoluble ingredients are also known in the art. For example, U.S. Patent No. 3,516,941 describes providing an aqueous solution of a water-insoluble ingredient to be encapsulated to a solution of water-soluble polymer in a reactor. The components are then subjected to high shear agitation that mixes the ingredient and polymer and breaks the ingredient down into very small bead-like capsules. A chemical catalyst is added to the mixture causing the polymer surrounds and forms a shell around each bead of the ingredient to be encapsulated. U.S. Patent No. 4,328,119 discloses encapsulating hydrophobic core material by depositing an anion-modified aminoaldehyde resin on the core material.

The elastomeric article may also comprise conventional latex compounding additives and modifiers, including surfactants, wetting agents, antioxidants, antistatic agents, antifoaming agents, anti-webbing agents, pH modifiers, viscosity modifiers, thickeners, plasticizers, pigments, fillers, vulcanizing agents, activators, crosslinkers, accelerators, and the like.

The elastomeric article can be packaged in such a way as to preserve its therapeutic properties during transportation and storage. In a preferred embodiment, the article is packaged in an inner wrap surrounded by an outer pack. These wraps may be made from paper, wax coated paper, polymer coated paper or polymeric sheet films that are well known in the art. The size of the inner wrap, after folding, is generally smaller than the outer pack for easy sealing. The pack is sealed such that a sufficient volume of a gas such as dry air is present to provide a cushioning effect to the inner wrap containing the article (a so-called "puffy pack"). In this manner, the inner wrap containing the article can move freely when the outer pack is held and shaken.

The powder-free elastomeric article can be prepared by a process comprising,
providing an elastomeric layer having an interior surface and an exterior surface;
applying a therapeutic fluid coating to the skin-contacting surface of the article;
drying the therapeutic fluid coating;
applying an anti-blocking coating to the therapeutic fluid coating;
drying at a temperature sufficient to form a liquid crystal gel and drying the gel to form a continuous solid film.

As noted above, one or more additional layers or coatings may be applied between the elastomeric layer and the gel coating, such as a slip coating.

When the elastomeric article is a glove, conventional dip methods may be employed, which include on-line processes for mass production of latex gloves. Generally, these methods involve forming the layers of the glove by, for example, dipping a pre-heated former into an aqueous coagulant, drying the coagulant on the former, dipping the former into a latex solution comprising natural and/or synthetic elastomers, and leaching the gelled latex in hot water. These steps can be repeated to build up elastomeric layers of the glove. If desired a second polymeric coating such as polyurethane may be applied to the skin contacting surface of the glove. The gloves are then cured. The outside surface of the gloves are then optionally treated chemically, such as by chlorination, to reduce tackiness. If chlorinated, the gloves are then washed.

The therapeutic and anti-block coatings can then applied to the cured elastomeric gloves. In specific embodiments, the elastomeric gloves are washed off-line prior to application of the therapeutic coating. This reduces therapeutic agent interfering materials. The gloves are then optionally dried with a blower or drier prior to application of the therapeutic and anti-block coatings by, for example, spraying tumbling or dipping. The therapeutic coating can then be coated onto the glove surface by spraying and drying in a dryer through one or more nozzles. The anti-block composition is then sprayed on the dried gloves with the therapeutic coating and dried at a temperature greater than the LCST temperature of the cellulose composition to facilitate liquid crystal film formation and dying the film to form a continuous solid film that covers the fragrant or skin nourishing gel coating preventing blocking of the interior surfaces of the glove. The final glove products are then inverted such that the gel coating is located on the interior side of the glove, and packaged in pairs, sterilized with gamma radiation, and cooled to room temperature.

In embodiments which utilize encapsulated agents, various methods of attaching or adhering encapsulated ingredients to a surface that are also known in the art may be incorporated in the processes described herein. Methods of attaching or adhering encapsulated ingredients to a surface are know in the art. For example, U.S. Patent No. 5,138,719 describes forming a solution of latex containing microcapsules and applying a layer of the latex to the surface of a glove by immersing the glove into the solution, followed by vulcanizing or hardening the formed layers. In one or more embodiments, the encapsulated agents may be bonded to the surface of a glove. U.S. Patent No. 4,898,633 describes coating the surface of a substrate with a binder resin having microcapsules dispersed therein. U.S. Patent Publication No. 2005/0066414 discloses bonding encapsulated antimicrobial agents to a surface using a polymer, for example, polyurethane.

Although the invention herein has been described with reference to particular embodiments, it is to be understood that these embodiments are merely illustrative of the principles and applications of the present invention. It will be apparent to those skilled in the art that various modifications and variations can be made to the method and apparatus of the present invention without departing from the scope of the invention. Thus, it is intended that the present invention include modifications and variations that are within the scope of the appended claims

## Claims

1. A powder-free glove, comprising:
an elastomeric layer having an interior surface and an exterior surface;
a therapeutic fluid coating disposed on the interior surface of the elastomeric layer; and
an anti-blocking film coating disposed on the therapeutic fluid coating.

2. The glove of claim 1, wherein the therapeutic fluid coating comprises a fragrance, a warming composition, a cooling composition, a skin-nourishing composition, or combinations thereof.

3. The glove of claim 1, wherein the elastomeric layer comprises a cured latex selected from the group consisting of natural latex, synthetic latex, and blends of natural and synthetic latex.

4. The glove of claim 3, wherein the synthetic latex is selected from the group consisting of polychloroprene, carboxylated acrylonitrile butadiene, polyisoprene, polyurethane, styrene-butadiene, and combinations thereof.

5. The glove of claim 1, wherein the therapeutic fluid coating comprises one or more fragrances.

6. The glove of claim 1, wherein the therapeutic fluid coating comprises a skin-conditioning agent that comprises a silicone composition.

7. The glove of claim 1, wherein the anti-blocking film coating comprises a cellulosic water-soluble polymer; and optionally, a water-insoluble occlusive moisturizer.

8. The glove of claim 7, wherein the cellulosic water-soluble polymer of the anti-blocking film coating is selected from the group consisting of hydroxylpropylcellulose (HPC), hydroxypropylmethylcellulose (HPMC), methyl cellulose (MC) polyvinylpyrrolidinone (PVP), and combinations thereof.

9. The glove of claim 7, wherein the water-soluble polymer has a low critical solution temperature greater than 40°C permitting dissolution of anti-blocking film coating by skin-generated moisture.

10. The glove of claim 7, wherein the water-insoluble occlusive moisturizer is aminofunctional siloxane, polydimethylsiloxane (dimethicone), oleyl erucate, or combinations thereof.

11. The glove of claim 1, wherein the anti-blocking film coating dissolves upon contact with moisture or is dislodged by rubbing action during use.

12. The glove of claim 1, wherein a therapeutic agent from the therapeutic fluid coating is released following dissolution or dislodgement of the anti-blocking film coating during use.

13. The glove of claim 1, wherein the therapeutic fluid coating exhibits substantially no loss of therapeutic activity following aging at 50°C for 8 weeks and 40°C for 6 months.

14. A process for preparing a powder-free glove comprising:
providing an elastomeric layer having an interior surface and an exterior surface;
applying a therapeutic fluid coating comprising one or more agents to the interior surface of the elastomeric layer;
drying the therapeutic fluid coating;
applying an anti-blocking film coating to the therapeutic fluid coating; and
drying the anti-blocking film coating.

15. The process of claim 14, wherein the anti-blocking film coating comprises a cellulosic water-soluble polymer; and the drying step comprises:
drying the anti-blocking film coating at a temperature greater than the low critical solution temperature of the cellulosic water-soluble polymer to form a liquid film, and
further drying of the liquid film to form a solid film coating covering said therapeutic fluid coating.

## Patentansprüche

1. Puderfreier Handschuh, Folgendes aufweisend:
eine Elastomerschicht mit einer Innenfläche und einer Außenfläche;
eine Beschichtung aus therapeutischem Fluid, die auf der Innenfläche der Elastomerschicht angeordnet ist; und
eine Antiblockierfilmbeschichtung, die auf der Beschichtung aus therapeutischem Fluid angeordnet ist.

2. Handschuh nach Anspruch 1, wobei die Beschichtung aus therapeutischem Fluid einen Duftstoff, eine wärmende Zusammensetzung, eine kühlende Zusammensetzung, eine hautpflegende Zusammensetzung oder Kombinationen von diesen umfasst.

3. Handschuh nach Anspruch 1, wobei die Elastomerschicht einen vulkanisierten Latex umfasst, der aus der Gruppe ausgewählt ist, die aus natürlichem Latex, synthetischem Latex und Mischungen aus natürlichem und synthetischem Latex besteht.

4. Handschuh nach Anspruch 3, wobei der synthetische Latex aus der Gruppe ausgewählt ist, die aus Polychloropren, carboxyliertem Acrylnitrilbutadien, Polyisopren, Polyurethan, Styrol-Butadien und Kombinationen von diesen besteht.

5. Handschuh nach Anspruch 1, wobei die Beschichtung aus therapeutischem Fluid einen oder mehrere Duftstoff/e umfasst.

6. Handschuh nach Anspruch 1, wobei die Beschichtung aus therapeutischem Fluid ein Rückfettungsmittel umfasst, das eine Silikonzusammensetzung umfasst.

7. Handschuh nach Anspruch 1, wobei die Antiblockierfilmbeschichtung ein von Cellulose abgeleitetes wasserlösliches Polymer und optional einen wasserunlöslichen, okklusiven Feuchtigkeitsspender umfasst.

8. Handschuh nach Anspruch 7, wobei das von Cellulose abgeleitete wasserlösliche Polymer der Antiblockierfilmbeschichtung aus der Gruppe ausgewählt ist, die aus Hydroxylpropylcellulose (HPC), Hydroxylpropylmethylcellulose (HPMC), Methylcellulose (MC), Polyvinylpyrrolidon (PVP) und Kombinationen von diesen besteht.

9. Handschuh nach Anspruch 7, wobei das wasserlösliche Polymer eine niedrige kritische Lösungstemperatur von über 40°C hat, die eine Auflösung der Antiblockierfilmbeschichtung durch hauterzeugte Feuchtigkeit zulässt.

10. Handschuh nach Anspruch 7, wobei es sich bei dem wasserunlöslichen okklusiven Feuchtigkeitsspender um aminofunktionelles Siloxan, Polydimethylsiloxan (Dimethicon), Oleylerucat oder Kombinationen von diesen handelt.

11. Handschuh nach Anspruch 1, wobei sich die Antiblockierfilmbeschichtung bei Kontakt mit Feuchtigkeit auflöst oder sich während des Gebrauchs durch Rubbelwirkung ablöst.

12. Handschuh nach Anspruch 1, wobei ein therapeutisches Mittel aus der Beschichtung aus therapeutischem Fluid während des Gebrauchs nach dem Auf- oder Ablösen der Antiblockierfilmbeschichtung freigesetzt wird.

13. Handschuh nach Anspruch 1, wobei die Beschichtung aus therapeutischem Fluid nach einer Alterung bei 50°C für 8 Wochen und 40°C für 6 Monate im Wesentlichen keinen Verlust an therapeutischer Wirksamkeit aufweist.

14. Prozess zum Herstellen eines puderfreien Handschuhs, Folgendes umfassend:
Bereitstellen einer Elastomerschicht mit einer Innenfläche und einer Außenfläche;
Aufbringen einer ein oder mehrere Mittel umfassenden Beschichtung aus therapeutischem Fluid auf die Innenfläche der Elastomerschicht;
Trocknen der Beschichtung aus therapeutischem Fluid;
Aufbringen einer Antiblockierfilmbeschichtung auf die Beschichtung aus therapeutischem Fluid; und
Trocknen der Antiblockierfilmbeschichtung.

15. Prozess nach Anspruch 14, wobei die Antiblockierfilmbeschichtung ein von Cellulose abgeleitetes wasserlösliches Polymer umfasst, und der Trocknungsschritt umfasst:
Trocknen der Antiblockierfilmbeschichtung bei einer Temperatur, die über der niedrigen kritischen Auflösungstemperatur des von Cellulose abgeleiteten wasserlöslichen Polymers liegt, um einen Flüssigkeitsfilm zu bilden, und
weiteres Trocknen des Flüssigkeitsfilms, um einen Feststofffilm zu bilden, der die Beschichtung aus therapeutischem Fluid bedeckt.

## Revendications

1. Gant exempt de poudre, comprenant :
une couche élastomère ayant une surface intérieure et une surface extérieure ;
un revêtement de fluide thérapeutique disposé sur la surface intérieure de la couche élastomère ; et
un revêtement de pellicule anti-adhérente disposé sur le revêtement de fluide thérapeutique.

2. Le gant de la revendication 1, sachant que le revêtement de fluide thérapeutique comprend une fragrance, une composition chauffante, une composition refroidissante, une composition nourrissante pour la peau, ou des combinaisons de celles-ci.

3. Le gant de la revendication 1, sachant que la couche élastomère comprend un latex durci sélectionné dans le groupe constitué par le latex naturel, le latex synthétique, et les mélanges de latex naturel et synthétique.

4. Le gant de la revendication 3, sachant que le latex synthétique est sélectionné dans le groupe constitué par le polychloroprène, le butadiène d'acrylonitrile carboxylé, le polyisoprène, le polyuréthane, le styrène-butadiène, et les combinaisons de ceux-ci.

5. Le gant de la revendication 1, sachant que le revêtement de fluide thérapeutique comprend une ou plusieurs fragrances.

6. Le gant de la revendication 1, sachant que le revêtement de fluide thérapeutique comprend un agent de regraissage de la peau qui comprend une composition de silicone.

7. Le gant de la revendication 1, sachant que le revêtement de pellicule anti-adhérente comprend un polymère cellulosique hydrosoluble ; et facultativement, un hydratant occlusif non hydrosoluble.

8. Le gant de la revendication 7, sachant que le polymère cellulosique hydrosoluble du revêtement de pellicule anti-adhérente est sélectionné dans le groupe constitué par l'hydroxylpropylcellulose (HPC), l'hydroxylpropylméthylcellulose (HPMC), la méthylcellulose (MC), le polyvinylpyrrolidinone (PVP), et les combinaisons de ceux-ci.

9. Le gant de la revendication 7, sachant que le polymère soluble dans l'eau a une température de solution critique basse supérieure à 40°C permettant la dissolution du revêtement de pellicule anti-adhérente par l'humidité générée par la peau.

10. Le gant de la revendication 7, sachant que l'hydratant occlusif non hydrosoluble est du siloxane aminofonctionnel, du polydiméthylsiloxane (diméthicone), de l'érucate oléylique, ou des combinaisons de ceux-ci.

11. Le gant de la revendication 1, sachant que le revêtement de pellicule anti-adhérente se dissout au contact de l'humidité ou est délogé par effet de frottement pendant l'utilisation.

12. Le gant de la revendication 1, sachant qu'un agent thérapeutique provenant du revêtement de fluide thérapeutique est libéré suite à la dissolution ou au délogement du revêtement de pellicule anti-adhérente pendant l'utilisation.

13. Le gant de la revendication 1, sachant que le revêtement de fluide thérapeutique ne présente sensiblement pas de perte d'activité thérapeutique suite au vieillissement à 50°C pendant 8 semaines et à 40°C pendant 6 mois.

14. Procédé d'élaboration d'un gant exempt de poudre comprenant :
la fourniture d'une couche élastomère ayant une surface intérieure et une surface extérieure ;
l'application d'un revêtement de fluide thérapeutique comprenant un ou plusieurs agents à la surface intérieure de la couche élastomère ;
le séchage du revêtement de fluide thérapeutique ;
l'application d'un revêtement de pellicule anti-adhérente au revêtement de fluide thérapeutique ; et
le séchage du revêtement de pellicule anti-adhérente.

15. Le procédé de la revendication 14, sachant que le revêtement de pellicule anti-adhérente comprend un polymère cellulosique hydrosoluble ; et l'étape de séchage comprend :
le séchage du revêtement de pellicule anti-adhérente à une température supérieure à la température de solution critique basse du polymère cellulosique hydrosoluble pour former une pellicule liquide, et
en outre le séchage de la pellicule liquide pour former un revêtement de pellicule solide recouvrant ledit revêtement de fluide thérapeutique.
